# EUROPEAN PATENT APPLICATION

(11) **EP 4 424 429 A1**
(43) Date of publication of application: **04.09.2024**
(21) Application number: 23159908.5
(22) Date of filing: 03.03.2023
(51) Int. Cl.: B05B 17/00, A61M 11/00, B06B 1/00

(54) **MEMBRANE UNIT FOR AN AEROSOL GENERATOR**

(71) Applicant: PARI Pharma GmbH, 82319 Starnberg (DE)
(72) Inventor: GALLEM, Thomas, 81371 München (DE); NEUNER, Michael, 90402 Nürnberg (DE)
(74) Representative: Hoffmann Eitle

(57) **Abstract**

The present disclosure relates to a membrane unit for an aerosol generator comprising a rigid substrate (11); a flexible circuit (50) having a conductive circuit pattern (52) formed on a flexible substrate (51); a membrane (100) supported by the flexible substrate (51) and having a plurality of through holes (121); and an oscillator (120) configured to vibrate the membrane (100), wherein the oscillator (120) is electrically interconnected with the conductive circuit pattern (52) of the flexible circuit (50), wherein the flexible circuit (50) comprises a membrane supporting portion (55) supporting the membrane (100), the membrane supporting portion (55) preferably having an annular shape, wherein the membrane supporting portion (55) is suspended via at least three spokes (56) to a surrounding portion (53).

## Description

### Technical field

The present disclosure relates to a membrane unit for an aerosol generator. The concerned aerosol generator is particularly used for aerosol therapy, wherein a liquid is nebulized/aerosolized by means of the membrane unit for being administered to a user (patient).

### Background

A membrane unit for this purpose is for example disclosed in WO 2006/084546 A1. This known membrane unit comprises a membrane having a plurality of through holes (apertures), an oscillator attached to the membrane and a flexible circuit comprising a flexible substrate having a conductive circuit pattern. The flexible substrate is configured for being fixed in the aerosol generator and for supporting the membrane and the oscillator.

Yet, it has been found difficult to accurately adjust the vibrational characteristics of the membrane and, therefore, allow for a reproducible nebulization process.

Even further, mounting of the membrane unit in the aerosol generator, particularly a releasable attachment, is difficult to be achieved due to the flexibility of the substrate.

In addition, there is a consistent need to further increase the integration scale of membrane units to even more cost-effectively produce aerosol generators.

### Summary

In view of the above it is an object to provide a membrane unit which may be improved with respect to the vibrational characteristics of the membrane so as to enable a reproducible nebulization process, allows easy attachment of the membrane unit to the aerosol generator and/or enables a high scale of integration.

This object is according to one aspect solved by a membrane unit as defined in claim 1. Embodiments of the present disclosure are defined in the dependent claims, the following description as well as the drawings.

Accordingly, a membrane unit for an aerosol generator is suggested. The unit is particularly defined by a rigid substrate, a flexible circuit, a membrane and an oscillator. Thus, the unit comprises (consist of) only four primary parts and has, hence, a simple and compact configuration.

The rigid substrate may be made from known circuit board materials, such as composite materials consisting of epoxy resin and fiberglass cloth. One specific example may be a composite material classified as FR4. Alternatively, the circuit board may also be made of plastic material such as polypropylene, polyamide or the like. In a specific example, the rigid substrate may be made of GRILAMID^{®}, PBT ASA, PP GF30 or Vectra (LCP). The rigid substrate may have a thickness in the range of 0.3mm to 3.0mm or preferred in the range of 0.5mm to 2.5mm or even more preferred in the range of 0.6mm to 1.5mm.

The flexible circuit has a conductive circuit pattern (first conductive circuit pattern) formed on a flexible substrate. The flexible circuit may also be referred to as flex circuit, flexible printed circuit board or flex print. The flexible substrate may be made from any electrically insulating material known for this purpose. In particular, the flexible substrate may consist of an insulating film, particularly a polymeric film on which the conductive circuit pattern is formed. In one particular example, a polyamide or polyimide base film is used as flexible substrate. In another particular example, a polyester or polyester base film is used as flexible substrate. The flexible substrate may have a thickness in the range of 50pm to 500pm, or preferred in the range of 70pm to 300pm, or even more preferred in the range of 90µm to 160pm. The flexible substrate may be built up in different layers, like a sandwich structure, for example the layers of the flexible substrate may have a first polymer layer (from 12pm to 50pm, for example 12pm, 25pm, or 50pm); a first adhesive layer, such as epoxy, (from 8µm up to 40pm); a copper layer (from 12pm to 70pm, for example 12pm, 17um, 35pm, or 70µm); a second adhesive layer, such as epoxy (from 8µm up to 40pm); and a second polymer layer (from 12pm to 50pm, for example 12pm, 25pm, or 50µm).

The rigidity of the rigid substrate is higher than the rigidity of the flexible substrate.

The rigid substrate may have a technical rigidity of 1,000MPa up to 15,000MPa. The technical rigidity of the rigid substrate may be measured via Rockwell hardness, ball indentation hardness, or compressive modulus (According to ISO 2039-1, ISO 604, or ISO 2039-2). The rigid substrate may be GRILAMID^{®}: (ball indentation hardness, ISO 2039-1, 120 MPa); Vectra (LCP) (compressive modulus, ISO 604, 14.000 MPa; or Rockwell hardness, ISO 2039-2, 71 M-Scal). The flexible substrate may be a Pyralux^{®} film (elastic stiffness constant 10,0 x 10¹⁰ N/m²).

On the other hand, the base material for the rigid substrate and for the flexible substrate may be the same and, thus, generally have the same technical rigidity. In any case, due to the lower thickness of flexible substrate as compared to the rigid substrate, the flexible substrate is more flexible than the rigid substrate. A thickness ratio of the rigid substrate to the flexible substrate may for example be at least 3, preferably at least 5, more preferably at least 6, even more preferably at least 9. For example the rigid substrate may have a thickness between 1.1 mm and 1.9 mm, as well as the flexible substrate may have a thickness between 0.1 mm and 0.4 µm.

The rigid substrate and the flexible substrate are affixed to each other. In one particular example, the rigid substrate and the flexible substrate are adhered (e.g. glued) to each other. Adhering the substrates together may include pressing the substrates together in a hot press at a pressure of approximately 5 MPa at a temperature of approximately 120°C to 140°C. Thus, at least the rigid substrate needs to be sufficiently heat resistant. In this context, the heat resistance of GRILAMID^{®} is 1,81 MPa at 160 C and that of Vectra^{®} (LCP) is 8 MPa at 216 C.

Yet, other processes for attaching the substrates are conceivable, such as molding, welding (including laser welding and ultrasonic welding), injection molding and/or hot forming, etc.

Moreover, the membrane is supported by the flexible substrate. In a particular example, the membrane is attached to the flexible substrate. The attachment may be realized by use of adhesive (glue) or any other technique, such as injection molding, form and/or force fitting, attachment using mechanical fasteners, thermoforming, welding (including laser welding, induction welding and resistance welding), salary and the like. Even further, the membrane may directly be attached to the flexible substrate or via any intermediate element, such as the oscillator or an electrically conductive film. In an alternative embodiment, the membrane is integrated into the flexible circuit, i.e. may be one piece with the flexible circuit in that the membrane supporting portion (described later) is configured as the membrane and has the plurality of through holes (apertures) (described later).

Further, the membrane may be made from any known material. The material may be electrically conductive. In a particular example, stainless steel is used for the membrane. However, other materials such as metal, steel, copper, titan, or even plastic are possible as well.

Furthermore, the membrane has a plurality of through holes (apertures). The through holes may preferably be manufactured by laser drilling or electroforming. However, other manufacturing processes may be used as well such as: puncturing, punching, micro-forming and the like. Upon vibrating the membrane, a liquid passes through the through holes from one side of the membrane to the other opposite side of the membrane whereby the liquid is nebulized.

For this purpose, the membrane unit further comprises the oscillator, which is configured to vibrate the membrane. The oscillator may for example be a piezoelectric element. The oscillator is electrically interconnected with the second conductive circuit pattern and/or first conductive circuit pattern.

In a particular example, one side of the oscillator may be attached (e.g. adhered) directly to the flexible substrate and, thereby, electrically interconnected to the first conductive circuit pattern and the other opposite side of the oscillator may be electrically contacted to the first conductive pattern via the membrane, the membrane being electrically interconnected to the first conductive pattern. Alternatively, the membrane may be directly attached (e.g. adhered) directly to the flexible substrate and, thereby, electrically interconnected to the first conductive circuit pattern and one side of the oscillator may be attached (e.g. adhered) directly to the membrane thereby also being electrically interconnected to the first conductive circuit pattern and the other opposite side of the oscillator may be electrically contacted to the first conductive pattern.

Due to the combined flexible and rigid properties of the subunit consisting of the flexible substrate and the rigid substrate, one has a plurality of variables to be adapted. Accordingly, a reproducible nebulization by correctly adjusting the vibrational characteristics of the membrane, easy and even releasable attachment of the membrane unit to the aerosol generator and the integration of further functions such as a plug (e.g. a USB plug as explained later) or electrical and/or electronic components resembling the controller of the aerosol generator or at least a part thereof (see later) into the membrane unit can be realized.

According to the invention and in order to provide beneficial vibrational characteristics of the membrane, the flexible circuit (flexible substrate) comprises a membrane supporting portion, which is according to an embodiment annular shaped, supporting the membrane. The membrane supporting portion is suspended via at least three spokes to a surrounding portion (frame portion) of the flexible circuit (flexible substrate). Due to the provision of the spokes, the membrane is more movably supported by the flexible substrate and the vibrational characteristics may easily be adjusted by changing the shape and/or dimension of the spokes. In this context, the spokes are integrated into the flexible substrate, i.e. the flexible substrate is an integrated one-piece part comprising the membrane supporting portion, the spokes and the surrounding portion (frame portion). A portion of first conductive circuit pattern may extend from the membrane supporting portion via the spokes to the surrounding portion.

Moreover, the first conductive circuit pattern may comprise a conductive pattern and the oscillator, having an annular shape, may be affixed (e.g. adhered) and electrically interconnected (connected) to the conductive pattern. The conductive pattern may be annular. Thus, a standard component (annular oscillator) may be used, and the electrical connection may easily be achieved by attachment of the oscillator to the annular conductive pattern.

The annular conductive pattern may be provided on the membrane supporting portion. Thus, the membrane supporting portion has a double function, namely supporting the oscillator as well as the membrane. In addition and because the membrane may preferably be made from electrically conductive material, such as stainless steel or the like, it is easy to achieve an electrical interconnection of the membrane and therefore the oscillator with the first conductive pattern (see foot described later).

The membrane may be electrically interconnected with the first conductive circuit pattern and is affixed (e.g. adhered) to one side of the oscillator and thereby electrically connected to that the side of the oscillator. Accordingly and as previously indicated, also the oscillator is electrically connected to the first conductive circuit pattern.

As previously described, the membrane may further comprise a foot for the electrical interconnection between the membrane and the first conductive circuit pattern, wherein the foot, or more particularly a contact surface thereof, is located in a different plane than the membrane, or more particularly a flange of the membrane surrounding an aerosolization portion having the through holes (apertures). The foot may be connected to the membrane or more particularly the flange via a web or arm. Furthermore, the foot may mechanically be connected to the flexible substrate by a welding process, such as resistance welding, laser welding, ultrasonic welding, by soldering, by clamping and/or by the use of conductive adhesive.

The rigid substrate may further comprise a recess, such as a blind hole, accommodating the electrical interconnection between the membrane and the first conductive circuit pattern. Thus, easy access to the interconnection between the membrane and the first conductive circuit pattern is enabled during the assembly process.

The recess may be filled with an electrically insulating material. Thus, after the connection between the foot and the first conductive circuit pattern has been realized, the connection is electrically insulated.

The rigid substrate may further comprise a frame, particularly an annular shaped or partly annular shaped frame, circumferentially surrounding the membrane. Accordingly, the frame may function as a cage protecting the membrane, e.g. when the membrane is mounted in the aerosol generator or during cleaning of the membrane.

The frame may comprise a plurality of radially extending ribs. In particular, a plurality of ribs may protrude from an inner circumference of the frame towards the center. A circle connecting the tips of the ribs may be dimensioned so as to center the membrane during the assembly process. Accordingly, the assembly process is facilitated. In addition, the number of ribs may correspond to the number of spokes of the flexible substrate (see above) and the ribs may be aligned with the spokes. As a result the ribs may be used for supporting the spokes to some extent and thereby adjust the vibrational characteristics of the membrane.

Even further, the flexible circuit may comprise an electrically insulating cover film partly covering the first conductive circuit pattern. Accordingly, only the relevant portions of the first conductive circuit pattern are accessible during the manufacturing process and any short-circuiting may, thereby, be prevented.

In one embodiment, the first conductive circuit pattern comprises electrical contact surfaces (which may also be referred to as pins) of a standardized plug/socket connection, particularly of a USB plug. In this connection two, four or more (and even an uneven number of) electrical contact surfaces may be provided. In the case of a USB configuration, the contact surfaces will be linear extending parallel to each other as seen in a direction perpendicular to a longitudinal direction of the linear contact surfaces.

According to one aspect, the first conductive circuit pattern may be formed on both of opposite surfaces of the flexible substrate. In an example, the electrical contact surfaces of the standardized plug/socket connection may be formed on a surface of the flexible substrate facing away from the rigid substrate. Other parts of the first conductive circuit pattern, such as the electrical connection to the membrane and/or the oscillator may be disposed on the opposite surface of the flexible substrate but being electrically connected to the electrical contact surfaces.

Alternatively, the first conductive circuit pattern may be formed on only one of opposite surfaces of the flexible substrate. Thereby, the manufacturing process of the flexible substrate/circuit can be simplified. In one example, the electrical contact surfaces of the standardized plug/socket connection may be formed on a surface of the flexible substrate facing the rigid substrate. In order to give access to the electrical contact surfaces, the rigid substrate may have cutouts (through holes) at a position corresponding to the electrical contact surfaces or a portion thereof. Consequently, electrical connection to the electrical contact surfaces may be realized via the cutouts.

The membrane unit may further have a rigid circuit having a conductive circuit pattern (second conductive circuit pattern) formed on the rigid substrate. The rigid substrate may in this case as well be referred to as circuit board.

By attaching the flexible substrate to the rigid substrate, the first and second conductive circuit patterns are electrically interconnected. Consequently, a combined rigid-and-flexible circuit board having a conductive circuit pattern is formed. In other words, a sub-unit is formed consisting of the flexible substrate and the rigid substrate creating a circuit board having flexible portions and rigid (non-flexible) portions.

In one embodiment, the second conductive circuit pattern comprises electrical contact surfaces of a standardized plug/socket connection, particularly of a USB plug. In this connection two, four or more (and even an uneven number of) electrical contact surfaces may be provided. In the case of a USB configuration, the contact surfaces will be linear extending parallel to each other as seen in a direction perpendicular to a longitudinal direction of the linear contact surfaces.

Moreover, the second conductive circuit pattern may have a secondary coil configured for providing an inductive coupling with a primary coil being connected with a controller of the aerosol generator for driving the oscillator. Thus, a wireless connection of the membrane unit to the controller and or a power supply may be realized. For more details in this regard, the skilled person is referred to EP 3 894 091 A1.

Moreover and to even further enhance the scale of integration, electrical and/or electronic components may be affixed to the rigid substrate and electrically interconnected with the second conductive pattern.

The electrical and/or electronic components may in a simple embodiment comprise components of an oscillating circuit connected to the oscillator for exciting the oscillator.

In particular, the electrical and/or electronic components are selected from the group consisting of inductors, capacitors, switches, power converters, semi-conductor components, RAM memories, ROM memories, light emitting elements (such as LEDs) and/or sensors (such as pressure sensors, flow sensors and/or temperature sensors.

Thus and in order to achieve the highest scale of integration, the electrical and/or electronic components embody a controller of the aerosol generator or at least a part thereof.

Even further, the electrical and/or electronic components may be encapsulated. The material of the encapsulation may be a biocompatible material such as TPE, polyimide, polyamide, an elastomer, adhesive (glue) or the like.

In order to allow attachment of the membrane unit in an aerosol generator in only one orientation, it may be beneficial that the rigid substrate is non-symmetrical (particularly relative to a longitudinal center axis passing the center of the membrane) comprising an indexing portion.

### Brief description of the drawing

Figure 1 shows a perspective top view of a membrane unit for an aerosol generator.
Figure 2 shows a perspective a bottom view of the membrane unit of figure 1.
Figures 3 A to E show consecutive steps of the manufacturing process of the membrane unit shown in figures 1 and 2.
Figure 4 A and B show an alternative configuration of the membrane unit for an aerosol generator in a perspective top view and a perspective a bottom view.
Figure 5 A and B show an even further alternative configuration of the membrane unit for an aerosol generator in a perspective top view and a perspective a bottom view.
Figure 6 shows a perspective top view of a membrane unit for another aerosol generator.
Figure 7 shows a perspective a bottom view of the membrane unit of figure 6;
Figures 8 A to E show consecutive steps of the manufacturing process of the membrane unit shown in figures 6 and 7;
Figures 9 A to C show a second embodiment of a membrane unit for an aerosol generator in A: a top view, B: a longitudinal cross section and C: a bottom view;
Figures 10 A to C show a third embodiment of a membrane unit for an aerosol generator in A: a top view, B: a bottom view and C: a modification of the third embodiment in a bottom view; and
Figures 11 A and B show simple examples of oscillating circuits.

### Detailed Description

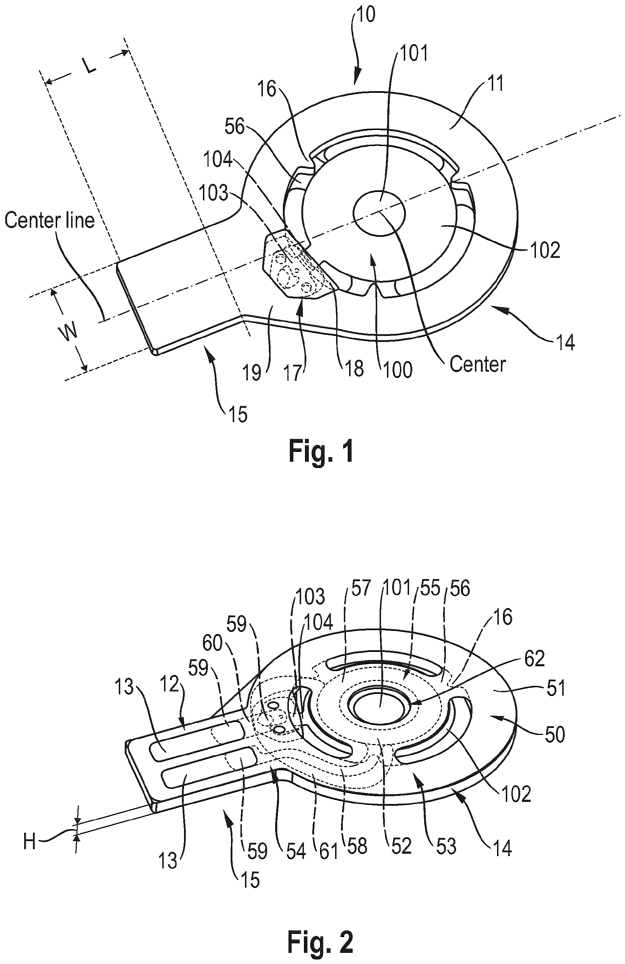

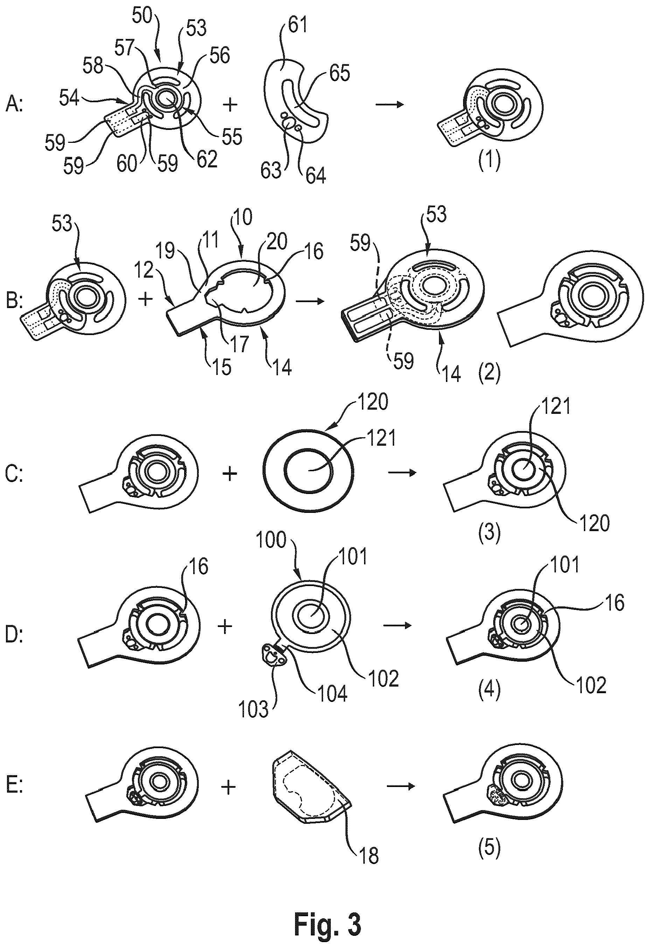

An embodiment of the present disclosure is shown in figures 1 to 3E. In this context, the flexible circuit 50 or more particularly the flexible substrate 51 in figure 2 has been shown in transparent so as to make the first conductive circuit pattern visible. Yet, it is to be understood that the flexible substrate does not need to be transparent even though it may be. In addition, the parts of the membrane unit are independently derivable from the consecutive steps of the manufacturing process in figures 3A to E. The flexible circuit 50 and an insulating cover film 61 are separately shown in the process step of figure 3A, whereas the rigid substrate 11 is derivable from the process step in figure 3B. The oscillator 120, the membrane 100 and the insulation 18 are respectively separately shown in the process steps of figures 3C, D and E.

The membrane unit shown in figures 1 and 2 comprises a rigid substrate 11, a flexible circuit 50, a membrane 100 and an oscillator 120. Thus, the membrane unit comprises four primary parts only. Thus, the disclosed membrane unit is compact, has a high scale of integration and is easy and inexpensive to manufacture and assemble.

The rigid substrate 11 may be made from any material including any known circuit board materials, such as composite materials consisting of epoxy resin and fiberglass cloth. One specific example may be a composite material classified as FR4. Alternatively, the circuit board may also be made of plastic material such as polypropylene, polyamide or the like. In one particular example, the rigid substrate 11 is made of GRILAMID^{®}, PBT ASA, PP GF30 or Vectra (LCP). In any case, the rigidity of the rigid substrate 11 is higher than the rigidity of the flexible substrate 51 of the flexible circuit 50 described later. The higher rigidity may in the first place be governed by a larger thickness of the rigid substrate 11 as compared to the flexible substrate 51 (see also below).

The rigid substrate 11 may have a thickness in the range of 0.3mm to 3.0mm or preferred in the range of 0.5mm to 2.5mm or even more preferred in the range of 0.6mm to 1.5mm.

In its most simple form, the rigid substrate 11 may be divided into a frame portion 14 and a plug portion 15.

The frame portion 14 may be annular having a through hole 20 with the plug portion 15 extending from the annular frame portion 14. The plug portion 15 may be aligned to the center of the frame portion 14 in that the center axis of the plug portion in a widthwise direction of the plug passes the center of the frame portion 14.

Moreover, the geometric shape of the plug portion 15 including the later described electrical contact surfaces 13 of the flexible circuit 50 (i.e. length (L), width (W), thickness (H) and arrangement/dimensioning of the pins/contact surfaces) in one embodiment resembles the configuration of a standard USB plug. Thus, the two conductive paths defining the electrical contact surfaces 13 (pins) may be gold-plated. Yet, it could also be more than the two conductive paths depending on the application. Two conductive paths are for example sufficient, if only a power supply is to be realized via the plug portion 15 (see later).

Thus, the membrane unit is directly or indirectly, via a standardized USB cable, connectable to a controller of the aerosol generator for controlling an oscillator 120 (see later).

The frame portion 14 may comprise a plurality of ribs 16 radially protruding from an inner circumference of the frame portion 14. In the shown embodiment, three of the ribs 16 are provided. The ribs 16 may serve for centering the membrane 100 during the manufacturing process and/or for supporting radial spokes 56 of flexible circuit 50 as will be explained further below.

Moreover, the rigid substrate 11 comprises a recess 17 which may be open towards the center of the frame portion 14, i.e. at the inner circumference of the frame portion 14. As will be explained later, the recess 17 serves for electrically interconnecting the membrane 100 to a portion (second conductive path 60) of the first conductive circuit pattern 52 of the flexible circuit 50 described earlier.

The recess 17 is filled with an electrically insulating material 18 to electrically insulate the connection between the membrane 100 and the first conductive circuit pattern 52.

Further, the rigid substrate 11 is relative to the centerline non-symmetric. In particular, it may comprise an indexing portion 19 on one side of the center axis. In the shown embodiment of figures 1 and 2, the indexing portion 19 is realized by a straight tangential portion extending from an outer circumference of the frame portion 14 to the plug portion 15. The opposite side of the rigid substrate 11 to the indexing portions 17 relative to the centerline has a curved recess between the frame portion 14 and the plug portion 15. As a result and if the aerosol generator has a recess for accommodating the membrane unit matching the shape of the rigid substrate 11, rigid substrate 11 and, hence, the membrane unit may only be accommodated in one orientation. Consequently, the membrane unit may not be wrongly mounted.

The flexible circuit 50 comprises a flexible substrate 51 and a (first) conductive circuit pattern 52 formed on the flexible substrate 51. The flexible circuit 50 may also be referred to as flex circuit, flexible printed circuit board or flex print. The flexible circuit 50 is fixed/attached to the rigid circuit 10. In one example, the flexible substrate 51 and the rigid substrate 11 are adhered to each other. Adhering the substrates together may include pressing the substrates together in a hot press at a pressure of approximately 5 MPa at a temperature of approximately 120°C to 140°C. Thus, at least the rigid substrate needs to be sufficiently heat resistant. In this context, the heat resistance of GRILAMID^{®} is 1,81 MPa at 160 C, that of PBT ASA is 1,8 MPa (DIN EN ISO 75-1) at 185 C (DIN EN ISO 306), that of PP GF30 is 45 MPa (0,45N/mm²) at 158 C (ISO EN 75-1/-2) and at 164 C° (DIN EN ISO 306) and that of Vectra^{®} (LCP) is 8 MPa at 216 C . Yet, also other processes for attaching the parts are conceivable such as welding, particularly ultrasonic welding and laser welding, the use of mechanical fasteners or the like.

The flexible substrate 51 may be made from any material. In particular, the flexible substrate 51 may consist of an insulating film, particularly a polymeric film on which the second conductive circuit pattern 52 is formed. In one particular example, a polyamide or polyimide base film is used as flexible substrate 51. In one particular example, a Pyralux^{®} film is used as flexible substrate 51. The flexible substrate 51 may have a thickness in the range of 50pm to 500pm, or preferred in the range of 70pm to 300pm, or even more preferred in the range of 90µm to 160pm. The flexible substrate may be built up in different layers, like a sandwich structure, for example the layers of the flexible substrate may have a first polymer layer (from 12pm to 50pm, for example 12pm, 25pm, or 50pm); a first adhesive layer, such as epoxy, (from 8µm up to 40pm); a copper layer (from 12pm to 70pm, for example 12pm, 17um, 35pm, or 70µm); a second adhesive layer, such as epoxy, (from 8µm up to 40pm); and a second polymer layer (from 12pm to 50pm, for example 12pm, 25pm, or 50µm).

As previously mentioned, due to the lower thickness of flexible substrate as compared to the rigid substrate, the flexible substrate is more flexible than the rigid substrate. A thickness ratio of the rigid substrate to the flexible substrate may for example be at least 3, preferably at least 5, more preferably at least 6.

The flexible substrate 51 comprises, in this embodiment, a frame portion 53, a plug portion 54 and a membrane supporting portion 55.

The frame portion 53 is annular in shape and surrounds the membrane supporting portion 55. The membrane supporting portion 55 may be annular shaped having a through hole 62 in its center. The frame portion 53 and the membrane supporting portion 55 are connected via spokes 56 constituted by the flexible substrate 51. The spokes 56 movably support or suspend the membrane supporting portion 55 relative to the remainder of the flexible substrate 51 (the frame portion 53 and the plug portion 54). In a particular embodiment, three of the spokes 56 are provided. In this context, the number of the spokes 56 may correspond to the number of the ribs 16, particularly if the ribs 16 serve for supporting the spokes 56. The spokes 56 particularly serve for vibrationally decoupling the vibratable membrane 100 from the rigid substrate 11. In this context, the spokes 56 may be straight radial webs as shown in the drawings. Yet, other shapes, such as an S-shape, are as well possible and may even advantageously support decoupling of the vibratable membrane 100 from the rigid substrate 11. Examples of different shapes of the spokes 56 are derivable from EP 1 386 672 B1.

In addition and as best visible from figure 2, the radial ribs 16 support the spokes 56 on one side. In this context, the ribs 16 support the spokes 56 on a side opposite to an aerosolization side of the membrane 100. Thus, the ribs 16 may be shaped and dimensioned so as to adjusting the vibrating characteristics of the membrane 100.

The first conductive circuit pattern 52 comprises a conductive pattern 57. As will be described later, the oscillator 120 may be attached or affixed to the conductive pattern 57. The conductive pattern 57 may be an annular conductive pattern 57.

The first conductive circuit pattern 52 further comprises two electrical contact surfaces 13. The first conductive circuit pattern 52 in this embodiment is formed on both of opposite surfaces of the flexible substrate 51. The electrical contact surfaces 13 may be formed on a first surface of the flexible substrate 51 opposite to a second surface of the flexible substrate 51 on which other portions of the first conductive circuit pattern 52 are formed. In particular, a first conductive path 58 of the first conductive circuit pattern 52 is formed on the second surface of the flexible substrate 51 connecting the conductive pattern 57 to one of the electrical contact surfaces 13 via a connecting portion at which the first conductive path 58 and the electrical contact surfaces 13 located on opposite side surfaces are electrically connected. The first conductive path 58 accordingly extends from the first conductive circuit pattern 52 via one of the spokes 56 to the electrical contact surface 13 on the plug portion 54.

A second conductive path 60 of the first conductive circuit pattern 52 is as well formed on the second surface of the flexible substrate 51. One of opposite ends of the second conductive path 60 is provided with a connection portion 59. The other of opposite ends of the second conductive path 60 forms another connection portion 59 at which the second conductive path 60 is connected to the other one of the electrical contact surface 13 located on opposite side surfaces are electrically connected. The second conductive path 60 serves for electrically interconnecting the membrane 100 and, hence, the opposite side of the oscillator 120 which is, as described later, in electrical contact with the membrane 100 to the other one of the electrical contact surfaces 13.

Moreover, an electrically insulating cover film or sheet 61 is provided (e.g. attached, such as adhered, or coated with an electrically insulating coating) on the flexible substrate 51 so as to electrically insulate and partly cover the first and second conductive paths 58 and 60 except for the conductive pattern 57 and the electrical contact surfaces 13. More particular, the cover film 61 should cover any portion of the second conductive circuit pattern 52 which would in the final membrane unit otherwise be exposed except for the electrical contact surfaces 13. In order to allow for the electrical connection of the foot 103 to the connecting portion 59 of the second conductive path 60, the cover film 61 has a first opening 63. For the mechanical connection of the foot 103 to the flexible substrate 51 second openings 64 may be provided in the cover film 61. Moreover and in order not to distort the vibrating characteristics adjusted via the spokes 56, a curved elongated hole 65 may be provided matching the elongated opening between adjacent spokes 56.

The cover film 61 may be made of any electrically insulating material, such as polyimide, polyamide, an elastomer, adhesive (glue) and so on.

The membrane unit further comprises an oscillator 120. The oscillator 120 may be a piezoelectric element. In one example, the oscillator 120 may be flat and/or annular shaped having a through hole 121 at its center. The oscillator 120 and the membrane supporting portion 55 of the flexible circuit 50 are dimensioned so as to match each other. For example, the outer diameter of the oscillator 120 may be equal to or slightly smaller/larger than the outer diameter of the membrane supporting portion 55. In an example, the outer diameter of the oscillator 120 may be equal to or slightly smaller than the outer diameter of the membrane supporting portion 55. Similar, the inner diameter of the membrane supporting portion 55 (the diameter of the through hole 62) may be substantially the same as that of the inner diameter of the oscillator 120 (the diameter of the through hole 121).

As indicated earlier, the oscillator 120 is attached, for example adhered, to the membrane supporting portion 55 of the flexible substrate 51 of the flexible circuit 50. In particular, the oscillator 120 is adhered to the conductive pattern 57 and, thereby, electrically contacted to the conductive pattern 57, The conductive pattern 57 may be annular. Hence, if an adhesive is used for attachment of the oscillator 120, the adhesive should be insulating the conductive pattern 57 and the oscillator 120, whereas the contact surfaces of the oscillator 120 and the conductive pattern 57 should be forming the electrical contact. For this reason, the oscillator 120 and the conductive pattern 57 should be pressed against each other during the curing process of the adhesive. As a result, one side of the oscillator 120 is electrically connected to the electrical contact surface 13 on the right in figure 2 via the conductive pattern 57 and the first conductive path 58. The conductive pattern 57 may be annular.

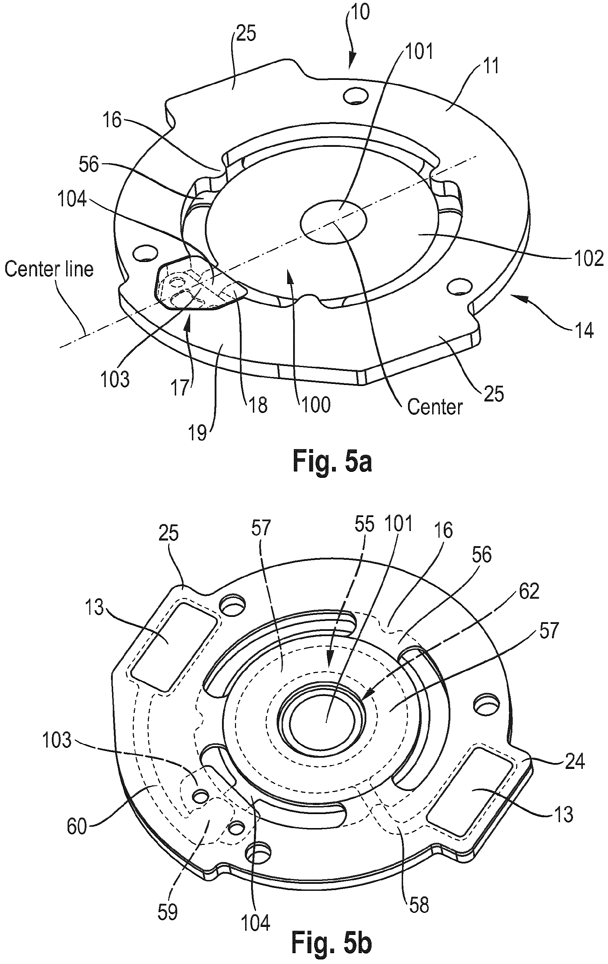

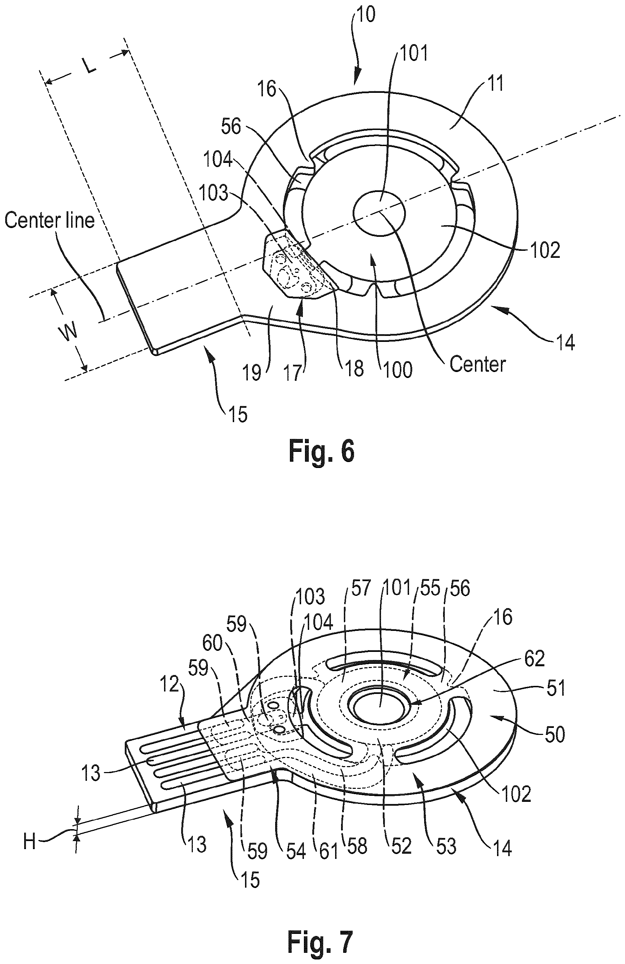

Moreover, the membrane unit comprises a membrane 100. The membrane 100 comprises an aerosolization portion 101 in which a plurality of through holes (apertures) are formed. A liquid to be nebulized (aerosolized) is fed to one side of the aerosolization portion 101 (upper side in figure 5) and nebulized through the through holes (apertures) to the opposite side of the aerosolization portion 101 (upper side in figure 6). The aerosolization portion 101 may be circular in size and be dimensioned so as to fit within the through hole 62 of the membrane supporting portion 55 and/or the through hole 121 of the oscillator 120.

Further, the membrane 100 comprises a flange portion 102 which may be substantially flat surrounding the aerosolization portion 101 radially extending therefrom. The flange portion 102 may serve for attaching the membrane 100 to the side of the oscillator 120 facing away from the flexible substrate 51. Again, the flange portion 102 may be adhered to the oscillator 120 preferably using an electrically insulating adhesive. For this reason, the membrane 100 and the oscillator 120 should be pressed against each other during the curing process of the adhesive. The flange portion 102 of the membrane 100 is forming the electrical contact.

Moreover, the flange portion 102 may with respect to the outer diameter be dimensioned so as to fit inside (to have a slightly smaller diameter than) a circle constructed by contacting the tips of all radial ribs 60 formed at the inner circumference of the frame portion 14 of the circuit board 11. Thus, the ribs 16 may serve for centering the membrane 100 during the assembly of the membrane unit.

Furthermore, a foot 103 may via a web (arm) 104 extend from the circumference of the flange portion 102. The foot 103 may have a flat contacting surface for being electrically contacted to the connecting portion 59 at one end of the second conductive path 60. Moreover, it may be beneficial that a flat (non-curved) plane corresponding to the flat contacting surface of the foot 103 differs from a flat (non-curved) plane corresponding to the radial extension of the flange portion 102. In one example, the planes may be parallel but distanced.

The membrane 100 may be made from any material known for this purpose, but in the present embodiment the material should be at least partly electrically conductive. In the particular example, stainless steel is used as material for the membrane 100. However, other materials such as metal, steel, copper, nickel, titan or even plastic are possible.

After attaching the membrane 100 to the oscillator 120, the foot 103 or more particularly its contact surface is electrically contacted to the connecting portion 59 of the second conductive path 60, whereby the other side of the oscillator 120 is electrically connected to the electrical contact surface 13 on the left in figure 2. For this purpose, a welding process, such as ultrasonic welding, resistance welding, laser welding or the like may be used. Other conceivable processes are soldering and/or the use of adhesive.

The connection is subsequently electrically insulated by the insulation 18 in the recess 17 as previously described. For this purpose, a thermomelt such as a technomelt, an Eccobond and/or a TPE embedding of a larger portion of the membrane unit are conceivable. Also combinations thereof are conceivable. Use of the material depends on the later application of the membrane unit. For example, if biocompatibility is a requirement, a biocompatible material may be used for insulation and for being disposed in the recess. Alternatively, an Eccobond may be filled in the recess 17 and a portion of the membrane unit be embedded in a TPE embedding covering the recess and portions of the membrane unit beyond the recess 17. For this purpose, the previously prepared membrane unit may for example be placed in a cavity and the insulating material may be gravity fed (poured) into the cavity without applying a pressure. Other insert molding techniques may however be applied as well.

In the depicted embodiment, the membrane 100 is an integrated one-piece part. Yet, it is conceivable that the membrane 100 consists of several parts such as an annular support and a membrane portion attached to the annular support, as for example described in EP 1 569 710 B1, EP 1 850 896 B1 or EP 2 437 896 B1.

In addition, it is also conceivable to integrate the membrane into the flexible circuit. For example, instead of providing a through hole 62 in the membrane supporting portion 55, one may provide the aerosolization portion 101 at the position of the through hole 62 of the flexible substrate 51. In this case, a separate electric connection will, however, be required to connect the oscillator 120 to the first conductive circuit pattern 12.

In the following, the assembly process of the previously described membrane unit is explained with reference to figure 3.

In a first step (figure 3A), the flexible circuit 50 is provided by applying the second conductive circuit pattern 52 onto a flexible substrate 51 (base film).

Subsequently an insulating cover film 61 is adhered to a side of the flexible substrate 51 having the first conductive circuit pattern 52 so as to cover exposed portions of the first conductive circuit pattern 52 except for the connecting portion 59 and the annular conductive pattern 57. Thus, the opening 63 is aligned to the connecting portion 59 of the second conductive path 60 and the elongated hole 65 is aligned with the elongated hole of the flexible substrate 51 between two adjacent spokes 56.

Accordingly, one obtains a flexible circuit 50 including the insulating cover film 61 (intermediate stage (1) product/flexible circuit unit).

During a second step (figure 3B), the rigid substrate 11 is provided.

Subsequently, the previously prepared intermediate stage (1) product is attached (adhered) to the rigid substrate 11. More particular, the flexible substrate 51 is with its frame portion 53 attached to the frame portion 14 of the rigid substrate 11 so that the first conductive circuit pattern 52 faces the rigid substrate 11. Thus, a support having rigid and flexible portions as well as electrically conductive paths is obtained (intermediate stage (2) product/rigid-and-flexible circuit unit).

In a third step (figure 3C), an oscillator 120 is provided and adhered to the side of the membrane supporting portion 55 of the flexible substrate 51 having the annular conductive pattern 57. Thereby, one side of the oscillator 120 is electrically connected to the annular conductive pattern 57 and an intermediate stage (3) product is obtained.

The fourth step (figure 3D) attaches a provided membrane 100 to the side of the oscillator 120 facing away from the flexible substrate 51. Thereby, the membrane 100 is electrically contacted to the other side of the oscillator 120. In this step, the flange portion 102 is adhered to the side of the oscillator 120, wherein in the center of the aerosolization portion 101 is aligned to the center of the through hole 121 of the oscillator 120. As previously indicated, the ribs 16 assist in centering the membrane 100 relative to the intermediate stage (3) product.

In addition, the foot 103 is with its contact surface placed on the connecting portion 59 of the second conductive path 60 located in the recess 17, whereby the foot 103 and, hence via the flange portion 102, the other side of the oscillator 120 is electrically connected to the second conductive path 60. In order to mechanically attach the foot 103 to the flexible substrate 51 a welding process, particularly ultrasonic welding, laser welding, resistance welding, soldering, the use of mechanical fasteners or the like is used.

The intermediate stage (4) product obtained in the fourth step is then further processed in the fifth step (figure 3E) by pouring (gravity feeding) a thermoplast, such as a technomelt, an Eccobond and/or a TPE into the recess 17 in order to form the insulation 18. For this purpose, the intermediate stage (4) product may be placed in a cavity and the cavity may be filled with the thermoplast. As a consequence, the interface between the foot 103 and the connecting portion 59 of the second conductive path 60 is electrically insulated by the insulation 18 and the final stage (5) product (membrane unit) is obtained.

In an additional optional step, the stage (5) product may partly be embedded in TPE (e.g. by insert molding) as it is to some extent described in WO 2009/13587181.

In the above-described embodiment, the membrane unit may via the plug portion 15 and the electrical contact surfaces 13 (for example pins) be directly or indirectly (using a cable) connected to a controller of the aerosol generator. Upon operation of the aerosol generator, the controller sends signals to the oscillator 120 via the electrical contact surfaces 13, the first and second conductive paths 58 and 60, the membrane 100 as well as the annular conductive pattern 57 so as to vibrate the membrane 100 and aerosolize a liquid applied to one side of the aerosolization portion 101 through the through holes to the opposite side of the aerosolization portion 101. In one advantageous embodiment, the electrical contact surfaces 13 resemble a standardized plug such as a USB plug and a standard cable or a standard USB socket may be used for connection to the controller. Thus, a highly integrated membrane unit may be obtained which can be manufactured at relatively low costs.

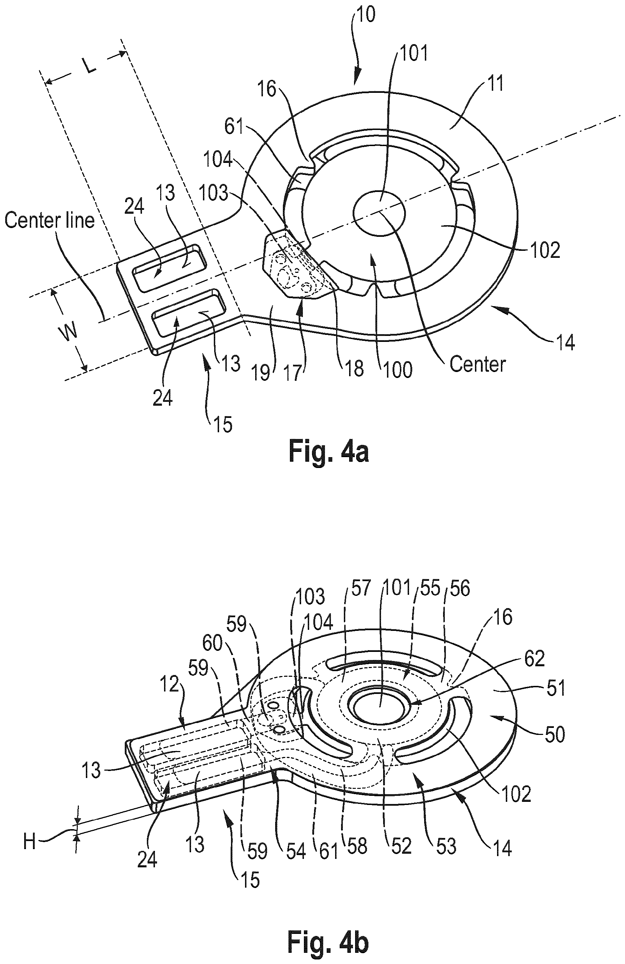

In the above embodiment, the electrical contact surfaces 13 are formed on the first surface of the flexible substrate 51, whereas the remainder of the first conductive circuit pattern 52 is formed on the second surface of the flexible substrate 51. Yet, it is also conceivable that the electrical contact surfaces 13 and the remainder of the first conductive circuit pattern 52 are formed on the second surface of the flexible substrate 51 as shown in figures 4A and 4B. Forming the entire first conductive circuit pattern 52 on only one surface (the second surface) of the flexible substrate 51 simplifies the manufacturing process of the flexible circuit 54. In order to give access to the electrical contact surfaces 13, the rigid substrate 11 comprises two cutouts 24 at locations corresponding to the electrical contact surfaces 13. Otherwise, this embodiment is similar to the embodiment described with respect to figures 1 to 3.

In another embodiment of the present disclosure, the plug portion 15 may be omitted and the electrical contact surfaces 13 may be formed on radially opposite sides as shown in figures 5A and 5B. Hence, the rigid substrate 11 and the flexible substrate 51 are basically circular. The electrical contact surfaces 13 are disposed on ears 25 protruding at radially opposite sides with respect to the through hole 62.

Moreover, the indexing portion 19, originally realized at the transition between the frame portion 53 and the plug portion 54, is embodied differently now extending from one of the ears 25 towards and beyond the recess 17, thus, dissolving the symmetry of the membrane unit.

It is to be understood that the configuration of the electrical contact surfaces 13 on the same side as the remainder of the first conductive circuit pattern 52 in combination with the cutouts 24 in the rigid substrate 11 as explained with respect to figures 4A and 4B may as well be embodied in the embodiment shown in figures 5A and 5B.

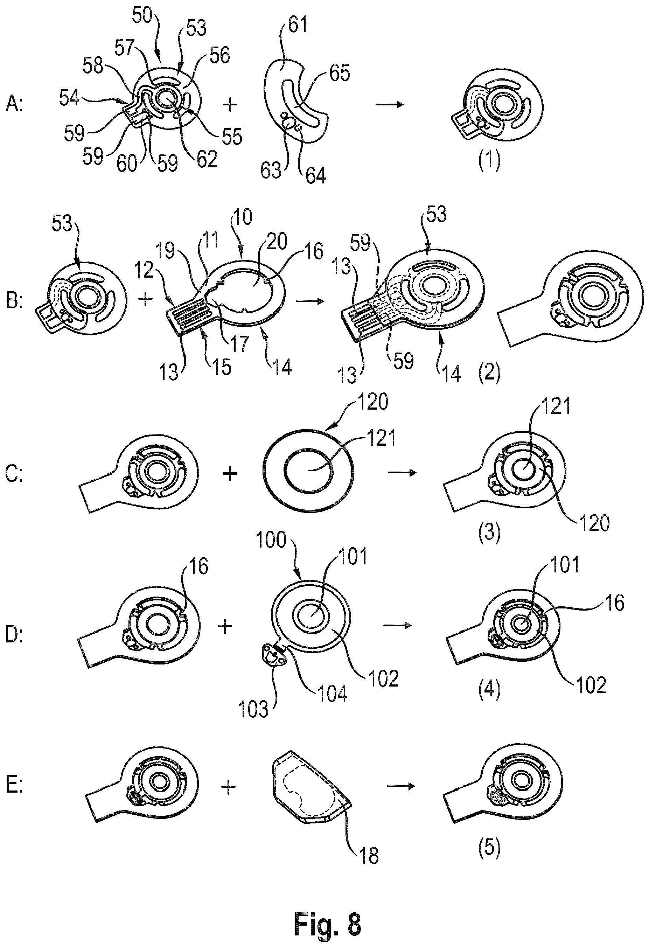

Another possible embodiment of the present disclosure is shown in figures 6 to 8E. In this context, the flexible circuit 50 or more particularly the flexible substrate 51 in figure 7 has been shown in transparent so as to make the first conductive circuit pattern visible. Yet, it is to be understood, that the flexible substrate does not need to be transparent even though it may be. In addition, the parts of the membrane unit are independently derivable from the consecutive steps of the manufacturing process in figure 8 A to E. The flexible circuit 50 and an insulating cover film 61 are separately shown in the process step of figure 8A, whereas the rigid circuit 10 is derivable from the process step in figure 8B. The oscillator 120, the membrane 100 and the insulation 18 are respectively separately shown in the process steps of figures 8C, D and E.

The membrane unit shown in figures 6 and 7 comprises a rigid circuit 10, a flexible circuit 50, a membrane 100 and an oscillator 120. Thus, the membrane unit comprises four primary parts only. Thus the disclosed membrane unit is compact, has a high scale of integration and is easy and inexpensive to manufacture and assemble.

The rigid circuit 10 comprises a circuit board (rigid substrate) 11. The circuit board 11 may be made from known circuit board materials, such as composite materials consisting of epoxy resin and fiberglass cloth. One specific example may be a composite material classified as FR4. Alternatively, the circuit board may also be made of plastic material such as polypropylene, polyamide or the like. In one particular example, the circuit board 11 is made of GRILAMID^{®} or Vectra (LCP). In any case, the rigidity of the circuit board 11 is higher than the rigidity of a later described flexible substrate 51 of the flexible circuit 50. The higher rigidity may in the first place be governed by a larger thickness of the circuit board 11 as compared to the flexible substrate 51 (see also below).

The circuit board 11 may have a thickness in the range of 0.3mm to 3.0mm or preferred in the range of 0.5mm to 2.5mm or even more preferred in the range of 0.6mm to 1.5mm.

The rigid circuit 10 further comprises a second conductive circuit pattern 12, which is, in this embodiment, constituted by four electrical contact surfaces 13 or conductive paths (printed circuit board tracks). Even more particular, the four electrical contact surfaces 13 are defined by longitudinal linear contact surfaces which are arranged parallel to each other perpendicular to their longitudinal extension.

In its most simple form, the rigid circuit 10 (the circuit board 11) may be divided into a frame portion 14 and a plug portion 15.

The frame portion 14 may be annular having a through hole 20 with the plug portion 15 extending from the annular frame portion 14. The plug portion 15 may be aligned to the center of the frame portion 14 in that the center axis of the plug portion in a widthwise direction of the plug passes the center of the frame portion 14.

Moreover, the geometric shape of the plug portion 15 including the electrical contact surfaces 13 (i.e. length (L), width (W), thickness (H) and arrangement/dimensioning of the pins/contact surfaces) in one embodiment resembles the configuration of a standard USB plug. Thus, the four conductive paths defining the electrical contact surfaces 13 (contact surfaces) may be gold-plated. Yet, it could also be less or more than the four conductive paths depending on the application. Two conductive paths are for example sufficient if only a power supply is to be realized via the plug portion 15 (see later).

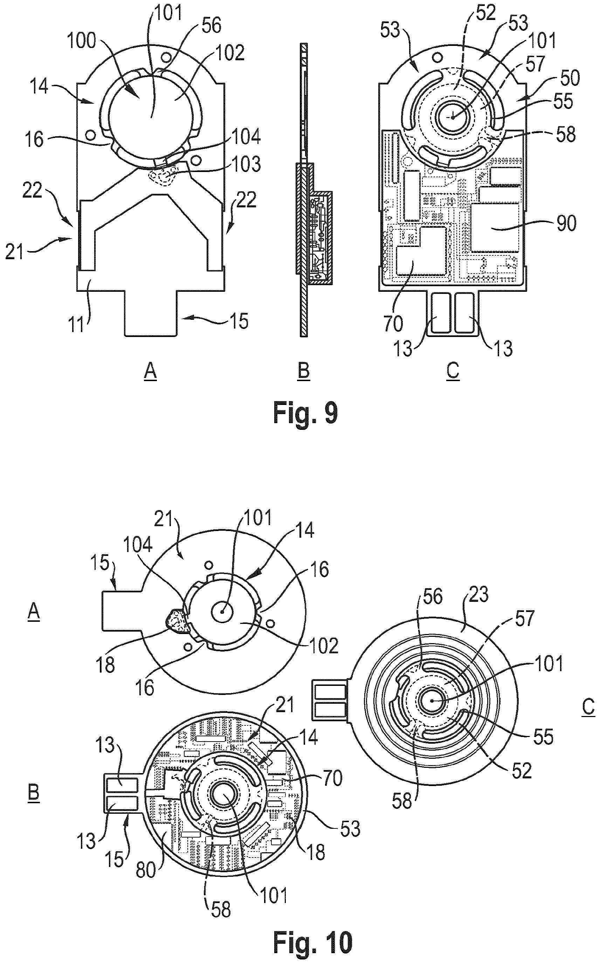

Thus, the membrane unit is directly or indirectly, via a standardized USB cable, connectable to a controller of the aerosol generator for controlling an oscillator 120 (see later) or a wall power supply to supply electrical and/or electronic components affixed to the circuit board 11 and electrically interconnected with the first conductive pattern 12 with power (see embodiments in figures 9 and 10).

The frame portion 14 may comprise a plurality of ribs 16 radially protruding from an inner circumference of the frame portion 14. In the shown embodiment, three of the ribs 16 are provided. The ribs 16 may serve for centering the membrane 100 during the manufacturing process and/or for supporting radial spokes 56 of flexible circuit 50 as will be explained further below.

Moreover, the circuit board 11 comprises a recess 17 which may be open towards the center of the frame portion 14, i.e. at the inner circumference of the frame portion 14. As will be explained later, the recess 17 serves for electrically interconnecting the membrane 100 to a portion (second conductive path 60) of the first conductive circuit pattern 52 of the flexible circuit 50 described earlier.

The recess 17 is filled with an electrically insulating material 18 to electrically insulate the connection between the membrane 100 and the first conductive circuit pattern 52.

Further, the circuit board 11 is relative to the centerline non-symmetric. In particular, it may comprise an indexing portion 19 on one side of the center axis. In the shown embodiment of figures 6 and 7, the indexing portion 19 is realized by a straight tangential portion extending from an outer circumference of the frame portion 14 to the plug portion 15. The opposite side of the circuit board 11 to the indexing portions 17 relative to the centerline has a curved recess between the frame portion 14 and the plug portion 15. As a result, and if the aerosol generator has a recess for accommodating the membrane unit matching the shape of the circuit board 11, the circuit board 11 (rigid circuit 10) and, hence, the membrane unit, may only be accommodated in one orientation. Consequently, the membrane unit may not be wrongly mounted.

The flexible circuit 50 comprises a flexible substrate 51 and a second conductive circuit pattern 52 formed on the flexible substrate 51. The flexible circuit 50 may also be referred to as flex circuit, flexible printed circuit board or flex print. The flexible circuit 50 is fixed/attached to the rigid circuit 10. In one example, the flexible substrate 51 and the circuit board 11 are adhered to each other. Adhering the substrates together may include pressing the substrates together in a hot press at a pressure of approximately 5 MPa at a temperature of approximately 120°C to 140°C. Thus, at least the rigid substrate needs to be sufficiently heat resistant. In this context, the heat resistance of GRILAMID^{®} is 1,81 MPa at 160 C and that of Vectra^{®} (LCP) is 8 MPa at 216 C. Yet, also other processes for attaching the parts are conceivable such as welding, particularly ultrasonic welding and laser welding, the use of mechanical fasteners or the like.

The flexible substrate 51 may be made from any material. In particular, the flexible substrate 51 may consist of an insulating film, particularly a polymeric film on which the second conductive circuit pattern 52 is formed. In one particular example, a polyamide or polyimide base film is used as flexible substrate 51. In one particular example, a Pyralux^{®} film is used as flexible substrate 51. The flexible substrate 51 may have a thickness in the range of 50pm to 500pm, or preferred in the range of 70pm to 300pm, or even more preferred in the range of 90µm to 160pm. The flexible substrate may be built up in different layers, like a sandwich structure, for example the layers of the flexible substrate may have a first polymer layer (from 12pm to 50pm, for example 12pm, 25pm, or 50pm); a first adhesive layer, such as epoxy, (from 8µm up to 40pm); a copper layer (from 12pm to 70pm, for example 12pm, 17um, 35pm, or 70µm); a second adhesive layer, such as epoxy, (from 8µm up to 40pm); and a second polymer layer (from 12pm to 50pm, for example 12pm, 25pm, or 50µm).

As previously mentioned, due to the lower thickness of flexible substrate as compared to the rigid substrate, the flexible substrate is more flexible than the rigid substrate. A thickness ratio of the rigid substrate to the flexible substrate may for example be at least 3, preferably at least 5, more preferably at least 6.

The flexible substrate 51 comprises, in this embodiment, a frame portion 53, a plug portion 54 and a membrane supporting portion 55.

The frame portion 53 is annular in shape and surrounds the membrane supporting portion 55. The membrane supporting portion 55 may be annular shaped having a through hole 62 in its center. The frame portion 53 and the membrane supporting portion 55 are connected via spokes 56 constituted by the flexible substrate 51. The spokes 56 movably support or suspend the membrane supporting portion 55 relative to the remainder of the flexible substrate 51 (the frame portion 53 and the plug portion 54). In a particular embodiment, three of the spokes 56 are provided. In this context, the number of the spokes 56 may correspond to the number of the ribs 16, particularly if the ribs 16 serve for supporting the spokes 56. The spokes 56 particularly serve for vibrationally decoupling the vibratable membrane 100 from the rigid circuit 10, particularly the rigid substrate (circuit board 11). In this context, the spokes 56 may be straight radial webs as shown in the drawings. Yet, other shapes, such as an S-shape, are as well possible and may even advantageously support decoupling of the vibratable membrane 100 from the rigid circuit 10. Examples of different shapes of the spokes 56 are derivable from EP 1 386 672 B1.

In addition and as best visible from figure 6, the radial ribs 16 support the spokes 56 on one side. In this context, the ribs 16 support the spokes 56 on a side opposite to an aerosolization side of the membrane 100. Thus, the ribs 16 may be shaped and dimensioned so as to adjusting the vibrating characteristics of the membrane 100.

The first conductive circuit pattern 52 comprises an annular conductive pattern 57. As will be described later, the oscillator 120 may be attached or affixed to the annular conductive pattern 57.

A first conductive path 58 of the first conductive circuit pattern 52 is formed on the flexible substrate 51 connecting the annular conductive pattern 57 and a connecting portion 59. The first conductive path 58 accordingly extends from the first conductive circuit pattern 52 via one of the spokes 56 to the connecting portion 59 on the plug portion 54. The connecting portion 59 is provided for electrically interconnecting the annular conductive pattern 57 and, hence, one side of the oscillator 120, to a portion of the second conductive circuit pattern 11 (in this embodiment to the two conductive paths (electrical contact surfaces 13) on the right in figure 7).

A second conductive path 60 of the first conductive circuit pattern 52 is as well formed on the flexible substrate 51. Opposite ends of the second conductive path 60 are provided with respective connection portions 59. The second conductive path 60 serves for electrically interconnecting the membrane 100 and, hence, the opposite side of the oscillator 120 which is, as described later, in electrical contact with the membrane 100, to a portion of the first conductive circuit pattern 11 (in this embodiment to the two conductive paths (electrical contact surfaces 13) on the left in figure 7).

Moreover, an electrically insulating cover film or sheet 61 is provided (e.g. attached, such as adhered, or coated with an electrically insulating coating) on the flexible substrate 51 so as to electrically insulate and partly cover the first and second conductive paths 58 and 60 except for the annular conductive pattern 57 and the connecting portions 59. More particular, the cover film 61 should cover any portion of the second conductive circuit pattern 52 which would in the final membrane unit otherwise be exposed. In order to allow for the electrical connection of the foot 103 to the connecting portion 59 of the second conductive path 60, the cover film 61 has a first opening 63. For the mechanical connection of the foot 103 to the flexible substrate 51 second openings 64 may be provided in the cover film 61. Moreover and in order not to distort the vibrating characteristics adjusted via the spokes 56, a curved elongated hole 65 may be provided matching the elongated opening between adjacent spokes 56.

The cover film 61 may be made of any electrically insulating material, such as polyimide, polyamide, an elastomer, adhesive (glue) and so on.

Upon attachment of the flexible circuit 50 to the rigid circuit 10, the connecting portions 59 are respectively electrically connected to the conductive paths (electrical contact surfaces 13) formed on the circuit board 11 as previously described.

The membrane unit further comprises an oscillator 120. The oscillator 120 may be a piezoelectric element. In one example, the oscillator 120 may be flat and/or annular shaped having a through hole 121 at its center. The oscillator 120 and the membrane supporting portion 55 of the flexible circuit 50 are dimensioned so as to match each other. For example, the outer diameter of the oscillator 120 may be equal to or slightly smaller/larger than the outer diameter of the membrane supporting portion 55. Similar, the inner diameter of the membrane supporting portion 55 (the diameter of the through hole 62) may be substantially the same as that of the inner diameter of the oscillator 120 (the diameter of the through hole 121).

As indicated earlier, the oscillator 120 is attached, for example adhered, to the membrane supporting portion 55 of the flexible substrate 51 of the flexible circuit 50. In particular, the oscillator 120 is adhered to the annular conductive pattern 57 and, thereby, electrically contacted to the annular conductive pattern 57. Hence, if an adhesive is used for attachment of the oscillator 120, the adhesive should be electrically conductive. As a result, one side of the oscillator 120 is electrically connected to the two conductive paths (electrical contact surfaces 13) on the right in figure 7 via the annular conductive pattern 57, the first conductive path 58 and the connecting portion 59.

Moreover, the membrane unit comprises a membrane 100. The membrane 100 comprises an aerosolization portion 101 in which a plurality of through holes (apertures) are formed. A liquid to be nebulized (aerosolized) is fed to one side of the aerosolization portion 101 (upper side in figure 6) and nebulized through the through holes (apertures) to the opposite side of the aerosolization portion 101 (upper side in figure 7). The aerosolization portion 101 may be circular in size and be dimensioned so as to fit within the through hole 62 of the membrane supporting portion 55 and/or the through hole 121 of the oscillator 120.

Further, the membrane 100 comprises a flange portion 102 which may be substantially flat surrounding the aerosolization portion 101 radially extending therefrom. The flange portion 102 may serve for attaching the membrane 100 to the side of the oscillator 120 facing away from the flexible substrate 51. Again, the flange portion 102 may be adhered to the oscillator 120 preferably using an electrically conductive adhesive.

Moreover, the flange portion 102 may with respect to the outer diameter be dimensioned so as to fit inside (to have a slightly smaller diameter than) a circle constructed by contacting the tips of all radial ribs 60 formed at the inner circumference of the frame portion 14 of the circuit board 11.

Thus, the ribs 16 may serve for centering the membrane 100 during the assembly of the membrane unit.

Furthermore, a foot 103 may via a web (arm) 104 extend from the circumference of the flange portion 102. The foot 103 may have a flat contacting surface for being electrically contacted to the connecting portion 59 at one end of the second conductive path 60. Moreover, it may be beneficial that a flat (non-curved) plane corresponding to the flat contacting surface of the foot 103 differs from a flat (non-curved) plane corresponding to the radial extension of the flange portion 102. In one example, the planes may be parallel but distanced.

The membrane 100 may be made from any material known for this purpose, but in the present embodiment, the material should be at least partly electrically conductive. In the particular example, stainless steel is used as material for the membrane 100. However, other materials such as metal, steel, copper, titan, or even plastic are possible.

After attaching the membrane 100 to the oscillator 120, the foot 103 or more particularly its contact surface is electrically contacted to the connecting portion 59 of the second conductive path 60, whereby the other side of the oscillator 120 is electrically connected to the two conductive paths (electrical contact surfaces 13) on the left in figure 7. For this purpose, a welding process, such as ultrasonic welding, resistance welding, laser welding or the like may be used. Other conceivable processes are soldering and/or the use of adhesive.

The connection is subsequently electrically insulated by the insulation 18 in the recess 17 as previously described. For this purpose, a thermomelt such as a technomelt, an Eccobond and/or a TPE embedding of a larger portion of the membrane unit are conceivable. Also combinations thereof are conceivable. Use of the material depends on the later application of the membrane unit. For example, if biocompatibility is a requirement, a biocompatible material may be used for insulation and for being disposed in the recess. Alternatively, an Eccobond may be filled in the recess 17 and a portion of the membrane unit be embedded in a TPE embedding covering the recess and portions of the membrane unit beyond the recess 17. For this purpose, the previously prepared membrane unit may for example be placed in a cavity and the insulating material may be gravity fed (poured) into the cavity without applying a pressure. Other insert molding techniques may however be applied as well.

In the depicted embodiment, the membrane 100 is an integrated one-piece part. Yet, it is conceivable that the membrane 100 consists of several parts such as an annular support and a membrane portion attached to the annular support, as for example described in EP 1 569 710 B1, EP 1 850 896 B1 or EP 2 437 896 B1.

In addition, it is also conceivable to integrate the membrane into the flexible circuit. For example, instead of providing a through hole 62 in the membrane supporting portion 55, one may provide the aerosolization portion 101 at the position of the through hole 62 of the flexible substrate 51. In this case, a separate electric connection will, however, be required to connect the oscillator 120 to the first conductive circuit pattern 12.

In the following, the assembly process of the previously described membrane unit is explained with reference to figure 8.

In a first step (figure 8A), the flexible circuit 50 is provided by applying the second conductive circuit pattern 52 onto a flexible substrate 51 (base film).

Subsequently an insulating cover film 61 is adhered to a side of the flexible substrate 51 having the first conductive circuit pattern 52 so as to cover exposed portions of the first conductive circuit pattern 52 except for the connecting portions 59 and the annular conductive pattern 57. Thus, the opening 63 is aligned to the connecting portion 59 of the second conductive path 60 and the elongated hole 65 is aligned with the elongated hole of the flexible substrate 51 between two adjacent spokes 56.

Accordingly, one obtains a flexible circuit 50 including the insulating cover film 61 (intermediate stage (1) product / flexible circuit unit).

During a second step (figure 8B), the rigid circuit 10 is provided by applying the second conductive circuit pattern 12 on the circuit board 11.

Subsequently, the previously prepared intermediate stage (1) product is attached (adhered) to the rigid circuit 10. More particular, the flexible substrate 51 is with its frame portion 53 attached to the frame portion 14 of the circuit board 11 so that the first conductive circuit pattern 52 faces the circuit board 11. Thereby the connecting portions 59 of the first and second conductive paths 58 and 60 are electrically connected to the conductive paths (electrical contact surfaces 13) formed on the flexible substrate 51. Thus, a rigid-and-flexible circuit board is obtained, i.e. a support having rigid and flexible portions as well as electrically conductive paths (intermediate stage (2) product / rigid-and-flexible circuit unit).

In a third step (figure 8C), an oscillator 120 is provided and adhered to the side of the membrane supporting portion 55 of the flexible substrate 51 having the annular conductive pattern 57. Thereby, one side of the oscillator 120 is electrically connected to the annular conductive pattern 57 and the intermediate stage (3) product is obtained.

The fourth step (figure 8D) attaches a provided membrane 100 to the side of the oscillator 120 facing away from the flexible substrate 51. Thereby, the membrane 100 is electrically contacted to the other side of the oscillator 120. In this step, the flange portion 102 is adhered to the side of the oscillator 120, wherein in the center of the aerosolization portion 101 is aligned to the center of the through hole 121 of the oscillator 120. As previously indicated, the ribs 16 assist in the centering the membrane 100 relative to the intermediate stage (3) product.

In addition, the foot 103 is with its contact surface placed on the connecting portion 59 of the second conductive path 60 located in the recess 17, whereby the foot 103 and, hence via the flange portion 102, the other side of the oscillator 120 is electrically connected to the second conductive path 60. In order to mechanically attach the foot 103 to the flexible substrate 51 a welding process, particularly ultrasonic welding, laser welding, resistance welding, soldering, the use of mechanical fasteners or the like is used.

The intermediate stage (4) product obtained in the fourth step is then further processed in the fifth step (figure 8E) by pouring (gravity feeding) a thermoplast, such as a technomelt, an Eccobond and/or a TPE into the recess 17 in order to form the insulation 18. For this purpose, the intermediate stage (4) product may be placed in a cavity and the cavity may be filled with the thermoplast. As a consequence, the interface between the foot 103 and the connecting portion 59 of the second conductive path 60 is electrically insulated by the insulation 18 and the final stage (5) product (membrane unit) is obtained.

In an additional optional step, the stage (5) product may partly be embedded in TPE (e.g. by insert molding) as it is to some extent described in WO 2009/13587181.

In the above-described embodiment, the membrane unit may via the plug portion 15 and the electrical contact surfaces 13 be directly or indirectly (using a cable) connected to a controller of the aerosol generator. Upon operation of the aerosol generator, the controller sends signals to the oscillator 120 via the electrical contact surfaces 13, the first and second conductive paths 58 and 60, the membrane 100 as well as the annular conductive pattern 57 so as to vibrate the membrane 100 and aerosolize a liquid applied to one side of the aerosolization portion 101 through the through holes to the opposite side of the aerosolization portion 101. In one advantageous embodiment, the electrical contact surfaces 13 resemble a standardized plug such as a USB plug and a standard cable or a standard USB socket may be used for connection to the controller. Thus, a highly integrated membrane unit may be obtained which can be manufactured at relatively low costs.

In order to even further increase the integrated state, additional components of the controller of the aerosol generator may be integrated into the membrane unit. One example in this regard is explained in more detail with reference to figures 9 to 11.

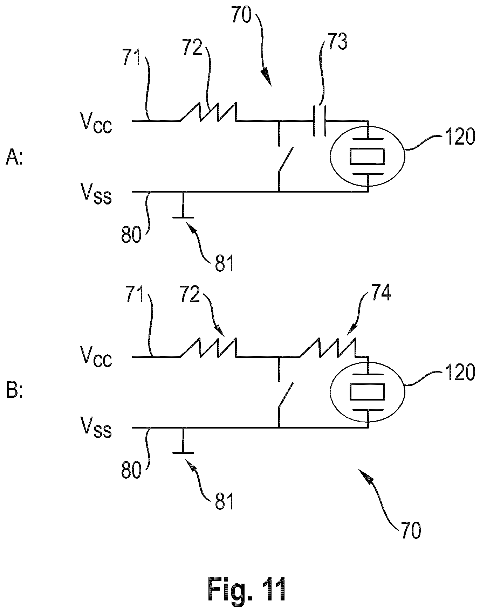

In its most simple form, an oscillating circuit is to be provided in the controller for isolating the oscillator 120 and, thereby vibrate the membrane 100 for aerosolization. Two examples of such oscillating circuits are shown in figures 11 A and B.

The oscillating circuit 70 in figure 11A comprises a first line 71 to be supplied with an input voltage Vcc. The first line 71 is connected to one side of the oscillator 120 and may comprise the previously described annular conductive pattern 57, the first conductive path and/or one or more of the electrical contact surface(-s) 13.

A second line 80 may be connected to the opposite side of the oscillator 120 and may comprise the previously described Flange portion 102 of the membrane 100, the web 104, the foot 103, the second conductive path 60 and/or one or more of the electrical contact surface(-s) 13. The second line 80 is earthed 81 or outputs an output voltage Vss.

In the first example in figure 11A, a first inductor 72 and a capacitor 73 are provided between the input voltage Vcc and the oscillator 120. In the second example in figure 11B the capacitor 73 is substituted by a second inductor 74.

Moreover, the lines 71 and 80 are connected via a switch 75 between the first inductor 72 and the capacitor 73/second inductor 74 so as to enable on/off switching of the oscillating circuit. The switch 75 may be realized without moving parts (for example capacitive).

In order to integrate such oscillating circuit 70 in the previously described membrane unit, it is suggested to extend the rigid circuit 10 or more particularly the circuit board 11 to further include an electronics portion 21 (see figures 9 and 10).

The electronics portion 21 may be formed by providing an additional portion between the frame portion 14 and the plug portion 15 as shown in figure 9 or by extending the radial dimension of the frame portion 14 as shown in figure 10.

In one example, an oscillating circuit 70 (the components thereof), e.g. the one described with respect to figure 11 A or B, is mounted on the circuit board 11 of the rigid circuit 10 and connected to the first conductive circuit pattern 12.

The electrical contact surfaces 13 (e.g. resembling Universal Serial Bus (USB) contact surfaces, like USB-1, USB-2, USB-3, USB-4, USB Typ A, USB Typ B, USB Typ C, USB Typ D, micro USB, or Lightning for Apple iPhone and so on) may be used to supply the controller with the required voltage of for example 5V, 7.5V or 12V DC. In the embodiment in figures 4 and 5 only to such electrical contact surfaces 13 are provided. As a consequence, one of the electrical contact surfaces 13 is connected to the first line 71 whereas the other electrical contact surface 13 is connected to the second line 80.

As previously described a power converter 90 may as well be mounted on the circuit board 11 of the rigid circuit 10 in order to convert the direct voltage (DC) into alternating voltage (AC). Thus, the voltage may be supplied directly via a USB cable inserted into a power plug plugged into a wall socket. Subsequently, the direct voltage (DC) is converted into alternating voltage (AC) by the power converter and subsequently supplied to the first line 71 of the oscillating circuit 70. The first line 71 then as in the first embodiment connects to one side of the oscillator 120 via the first conductive path 58 and the annular conductive pattern 57. The output voltage is then fed back via the second line 18 connected to the other side of the oscillator 120 has than the previous embodiment via the membrane 100, particularly the front portion 102, the web 104 and the foot 103 to the other of the electrical contact surfaces 13.

In the manufacturing process, the electronic components such as the oscillating circuit 70 and the power converter 90 or its components are soldered to the circuit board 11 of the rigid circuit and the so prepared rigid circuit 10 is provided in the second process step (figure 8B) explained above.

In addition in the fifth step in which the intermediate stage (4) product is placed in a cavity for providing the insulation 18 is not only the connection between the foot 103 and the connecting portion 59 which is electrically insulate but also the electronics portion and the electronic components disposed thereon. In this context, it may be beneficial to provide cutouts that enable flowing of the insulating material from one side of the circuit board 11 to the other side of the circuit board 11.

Finally and as shown in figure 10C, the electronics portion may further be embedded in TPE as described with respect to the first embodiment.

In the embodiment in Figures 9 and 10, one may achieve an even larger scale integration, because part of the usually external controller of the aerosol generator or even the entire controller may be integrated in the membrane unit further simplifying the structure and reducing the manufacturing costs of the aerosol generator as a whole.

### Reference numerals

- 10: rigid circuit
- 11: rigid substrate (circuit board)
- 12: second conductive circuit pattern
- 13: electrical contact surface (pin)
- 14: frame portion
- 15: plug portion
- 16: rib
- 17: recess
- 18: insulation
- 19: indexing portion
- 20: through hole
- 21: electronics portion
- 22: cutout
- 23: TPE Embedding
- 24: cutout
- 25: ear
- 50: flexible circuit
- 51: flexible substrate
- 52: first conductive circuit pattern
- 53: frame portion
- 54: plug portion
- 55: membrane supporting portion
- 56: spoke
- 57: annular conductive pattern
- 58: first conductive path
- 59: connecting portion
- 60: second conductive path
- 61: insulating cover film
- 62: through hole
- 63: first opening
- 64: second opening
- 65: elongated hole
- 70: oscillating circuit
- 71: first line
- 72: first inductor
- 73: capacitor
- 74: second inductor
- 75: switch
- 80: second line
- 81: earthing
- 90: power converter
- 100: membrane
- 101: aerosolization portion
- 102: flange portion
- 103: foot
- 104: web
- 120: oscillator
- 121: through hole

## Claims

1. Membrane unit for an aerosol generator comprising:
a rigid substrate (11);
a flexible circuit (50) having a conductive circuit pattern (52) formed on a flexible substrate (51);
a membrane (100) supported by the flexible substrate (51) and having a plurality of through holes (121); and
an oscillator (120) configured to vibrate the membrane (100), wherein the oscillator (120) is electrically interconnected with the conductive circuit pattern (52) of the flexible circuit (50), wherein the flexible circuit (50) comprises a membrane supporting portion (55) supporting the membrane (100), the membrane supporting portion (55) preferably having an annular shape, wherein the membrane supporting portion (55) is suspended via at least three spokes (56) to a surrounding portion (53).

2. Membrane unit according to claim 1, wherein the conductive circuit pattern (52) of the flexible circuit (50) comprises a conductive pattern (57), preferably provided on the membrane supporting portion (55), and the oscillator (120), having an annular shape, is affixed and electrically connected to the conductive pattern (57).

3. Membrane unit according to any one of the preceding claims, wherein the membrane (100) is electrically interconnected with the conductive circuit pattern (52) of the flexible circuit (50) and is affixed and thereby electrically connected to the oscillator (120).

4. Membrane unit according to claim 3, wherein the membrane (100) comprises a foot (103) for the electrical interconnection between the membrane (100) and the conductive circuit patterns (52) of the flexible circuit (50), wherein the foot (103) is preferably located in a different plane than the membrane (100).

5. Membrane unit according to any one of claims 3 and 4, wherein the rigid substrate comprises a recess (17) accommodating the electrical interconnection between the membrane (100) and the conductive circuit patterns (52) of the flexible circuit (50).

6. Membrane unit according to claim 5, wherein the recess (17) is filled with an electrically insulating material.

7. Membrane unit according to any one of the preceding claims, wherein the rigid substrate (11) comprises a frame (14), preferably having an annular shape, circumferentially surrounding the membrane (100).

8. Membrane unit according to claim 7, wherein the frame (14) comprises a plurality of radially protruding ribs (16).

9. Membrane unit according to any one of the preceding claims, wherein the flexible circuit (50) comprises an electrically insulating cover film (61) partly covering the conductive circuit pattern (52) of the flexible circuit (50).

10. Membrane unit according to any one of the preceding claims, wherein the conductive circuit pattern (52) of the flexible circuit (50) comprises contact surfaces (13) of an electrical plug/socket connection, optionally of a standardized electrical plug/socket connection, preferably of a USB plug.

11. Membrane unit according to any one of claims 1 to 10, wherein the conductive circuit pattern of the flexible circuit (50) is formed on both of opposite surfaces of the flexible substrate.

12. Membrane unit according to any one of claims 1 to 10, wherein the conductive circuit pattern of the flexible circuit (50) is formed on only one of opposite surfaces of the flexible substrate.

13. Membrane unit according to any one of claims 1 to 12, further comprising a rigid circuit (10) having a conductive circuit pattern (12) formed on the rigid substrate (11), wherein the rigid substrate (11) and the flexible substrate (51) are affixed to each other electrically interconnecting the conductive circuit pattern (12) of the rigid circuit (10) and the conductive circuit pattern (52) of the flexible circuit (50).

14. Membrane unit according claim 13, wherein the conductive circuit pattern (12) of the rigid circuit (10) comprises electrical contact surfaces (13) of a standardized plug/socket connection, particularly of a USB plug.

15. Membrane unit according to Claim 13 or 14, wherein the conductive circuit pattern (12) of the rigid circuit (10) has a secondary coil configured for providing an inductive coupling with a primary coil being connected with a controller of the aerosol generator for driving the oscillator (120).

16. Membrane unit according to any one of claims 13 to 15, wherein electrical and/or electronic components are affixed to the rigid substrate (11) and electrically interconnected with the conductive pattern (12) of the rigid circuit (10).

17. Membrane unit according to claim 16, wherein the electrical and/or electronic components comprise at least one of the group consisting of components of an oscillating circuit (70) connected to the oscillator (120) for exciting the oscillator (120).

18. Membrane unit according to claim 16 or 17, wherein the electrical and/or electronic components are selected from the group consisting of inductors (72, 74), capacitors (73), switches (75), power converters (90), semi-conductor components, RAM memories, ROM memories, light emitting elements and/or sensors.

19. Membrane unit according to any one of claims 16 to 18, wherein the electrical and/or electronic components embody a controller of the aerosol generator or a part thereof.

## Amended claims

### Amended claims in accordance with Rule 137(2) EPC.

1. Membrane unit for an aerosol generator comprising:
a rigid substrate (11);
a flexible circuit (50) having a conductive circuit pattern (52) formed on a flexible substrate (51);
a membrane (100) supported by the flexible substrate (51) and having a plurality of through holes (121); and
an oscillator (120) configured to vibrate the membrane (100), wherein the oscillator (120) is electrically interconnected with the conductive circuit pattern (52) of the flexible circuit (50), wherein the flexible circuit (50) comprises a membrane supporting portion (55) supporting the membrane (100), the membrane supporting portion (55) preferably having an annular shape, **characterized in that** the membrane supporting portion (55) is suspended via at least three spokes (56) to a surrounding portion (53) of the flexible circuit (50).

2. Membrane unit according to claim 1, wherein the conductive circuit pattern (52) of the flexible circuit (50) comprises a conductive pattern (57), preferably provided on the membrane supporting portion (55), and the oscillator (120), having an annular shape, is affixed and electrically connected to the conductive pattern (57).

3. Membrane unit according to any one of the preceding claims, wherein the membrane (100) is electrically interconnected with the conductive circuit pattern (52) of the flexible circuit (50) and is affixed and thereby electrically connected to the oscillator (120).

4. Membrane unit according to claim 3, wherein the membrane (100) comprises a foot (103) for the electrical interconnection between the membrane (100) and the conductive circuit patterns (52) of the flexible circuit (50), wherein the foot (103) is preferably located in a different plane than the membrane (100).

5. Membrane unit according to any one of claims 3 and 4, wherein the rigid substrate comprises a recess (17) accommodating the electrical interconnection between the membrane (100) and the conductive circuit patterns (52) of the flexible circuit (50).

6. Membrane unit according to claim 5, wherein the recess (17) is filled with an electrically insulating material.

7. Membrane unit according to any one of the preceding claims, wherein the rigid substrate (11) comprises a frame (14), preferably having an annular shape, circumferentially surrounding the membrane (100).

8. Membrane unit according to claim 7, wherein the frame (14) comprises a plurality of radially protruding ribs (16).

9. Membrane unit according to any one of the preceding claims, wherein the flexible circuit (50) comprises an electrically insulating cover film (61) partly covering the conductive circuit pattern (52) of the flexible circuit (50).

10. Membrane unit according to any one of the preceding claims, wherein the conductive circuit pattern (52) of the flexible circuit (50) comprises contact surfaces (13) of an electrical plug/socket connection, optionally of a standardized electrical plug/socket connection, preferably of a USB plug.

11. Membrane unit according to any one of claims 1 to 10, wherein the conductive circuit pattern of the flexible circuit (50) is formed on both of opposite surfaces of the flexible substrate.

12. Membrane unit according to any one of claims 1 to 10, wherein the conductive circuit pattern of the flexible circuit (50) is formed on only one of opposite surfaces of the flexible substrate.

13. Membrane unit according to any one of claims 1 to 12, further comprising a rigid circuit (10) having a conductive circuit pattern (12) formed on the rigid substrate (11), wherein the rigid substrate (11) and the flexible substrate (51) are affixed to each other electrically interconnecting the conductive circuit pattern (12) of the rigid circuit (10) and the conductive circuit pattern (52) of the flexible circuit (50).

14. Membrane unit according claim 13, wherein the conductive circuit pattern (12) of the rigid circuit (10) comprises electrical contact surfaces (13) of a standardized plug/socket connection, particularly of a USB plug.

15. Membrane unit according to Claim 13 or 14, wherein the conductive circuit pattern (12) of the rigid circuit (10) has a secondary coil configured for providing an inductive coupling with a primary coil being connected with a controller of the aerosol generator for driving the oscillator (120).

16. Membrane unit according to any one of claims 13 to 15, wherein electrical and/or electronic components are affixed to the rigid substrate (11) and electrically interconnected with the conductive pattern (12) of the rigid circuit (10).

17. Membrane unit according to claim 16, wherein the electrical and/or electronic components comprise at least one of the group consisting of components of an oscillating circuit (70) connected to the oscillator (120) for exciting the oscillator (120).

18. Membrane unit according to claim 16 or 17, wherein the electrical and/or electronic components are selected from the group consisting of inductors (72, 74), capacitors (73), switches (75), power converters (90), semi-conductor components, RAM memories, ROM memories, light emitting elements and/or sensors.

19. Membrane unit according to any one of claims 16 to 18, wherein the electrical and/or electronic components embody a controller of the aerosol generator or a part thereof.
